# EUROPÄISCHE PATENTANMELDUNG

(11) **EP 1 349 099 A2**
(43) Veröffentlichungstag der Anmeldung: **01.10.2003**
(21) Anmeldenummer: 03005633.7
(22) Anmeldetag: 12.03.2003
(51) Int. Cl.: G06F 19/00

(54) **Verfahren zur automatisierten Erfassung von Patienten-Aktionen**

(30) Priorität: 25.03.2002 DE 10213283
(71) Anmelder: SIEMENS AKTIENGESELLSCHAFT, 80333 München (DE)
(72) Erfinder: Abraham-Fuchs, Klaus, 91058 Erlangen (DE); Rumpel, Eva, Dr., 91052 Erlangen (DE); Tietze, Daniel, 91080 Spardorf (DE)

(57) **Zusammenfassung**

System zur automatisierten Erfassung von Patienten-Aktionen, beispielsweise im Zuge einer telemedizinischen Versorgung oder im Zuge klinischer Studien zur Güte einer Therapieform oder zur Erprobung der Wirksamkeit von Arzneimitteln, wobei die hierbei verwendeten medizinischen Geräte oder Einrichtungen mit Erfassungseinrichtungen zur Identifikation des Patienten versehen sind, um die bei der Benutzung der Geräte oder Hilfsmittel anfallenden Daten automatisch dem betreffenden Patienten zuzuordnen.

## Beschreibung

Die Erfindung bezieht sich auf ein Verfahren zur automatisierten Erfassung von Patienten-Aktionen, beispielsweise im Zuge einer telemedizinischen Versorgung oder im Zuge klinischer Studien zur Güte einer Therapieform oder zur Erprobung der Wirksamkeit von Arzneimitteln.

Ein großes Problem bei der Therapie von Patienten liegt darin, dass oft nicht geprüft werden kann, wie genau sich der Patient an die ärztlichen Verschreibungen hält (sogenannte Compliance), d. h. ob und wann er seine Medikamente einnimmt oder z. B. seinen Blutzucker bestimmt oder wie oft und wie langen er die ihm verordneten Übungen durchführt. Diese Informationen sind jedoch äußerst wichtig, wenn es darum geht, die Güte einer Therapieform, oder z. B. die Wirksamkeit eines neuen Medikamentes, zu beurteilen. So sind etwa bei der Einführung neuer Medikamente klinische Studien vorgeschrieben, die eine um 10 bis 20 % bessere Wirkung gegenüber einem bestehenden Präparat oder einem Placebo nachweisen. Dazu sind-aufgrund der hohen Variabilität der Messwerte - sehr große Teilnehmerzahlen erforderlich. Die Pharmaindustrie geht davon aus, dass 30 bis 40 % der Variabilität der Messwerte auf mangelnde Standardisierung der Rahmenbedingungen zurückzuführen ist: Medikamente werden nicht oder nicht pünktlich eingenommen, Messwerte zu verschiedenen Zeitpunkten und/oder auf die unterschiedlichste Weise erhoben etc. Ähnliches gilt für Studien zur Bewertung von Therapieergebnissen (sogenannte Outcome-Analyse), wenn nicht bekannt ist, wie genau sich die einzelnen Patienten an die Vorgaben gehalten haben. Eine vergleichbare Problemstellung ergibt sich schließlich auch bei Patienten, die mit neuartigen telemedizinischen Versorgungsformen betreut werden.

Bisher wurde der Mangel an Standardisierung durch eine zeitund kostenintensive Erhöhung der Probantenzahl ausgeglichen. Derartige Studien werden auch heute noch überwiegend papierbasiert erhoben, d. h. es werden Frage- und/oder Erhebungsbögen ausgefüllt, gesammelt und schließlich zentral ausgewertet. Eine Überprüfung der Richtigkeit der Angaben ist - wenn überhaupt - meist nur in Form von Plausibilitätskontrollen möglich.

Die vorstehend angesprochenen Probleme konnte auch ein Gerät zur Registrierung von Befindlichkeitswerten nicht lösen, wie es in der DE 196 14 255 C1 beschrieben ist. Dort geht es lediglich darum, dass der Patient ein mobiles Gerät mit sich führt, in das er jeweils Daten über Medikamenteneinnahme, Befindlichkeit usw. eindrückt. Ob ein Patient aber überhaupt und wenn ja über welche Zeitdauer ein bestimmtes Therapiegerät aufgesucht und benutzt hat, oder ob er die Tabletten eingenommen hat oder nicht, lässt sich mithilfe dieses Testgeräts nur bei sehr sorgfältigen Patienten ermitteln. Wenn der Patient die Tabletten einnimmt, ohne dass er sein Gerät bedient, lässt sich dies nicht feststellen. Entscheidend ist aber, dass es hierbei nur darum geht, dass ein Patient seine Daten in ein bei ihm befindliches Aufzeichnungsgerät eingibt. Bei der automatisierten Erfassung von Patientenaktionen geht es aber darum, dass eine Vielzahl von Geräten und Einrichtungen benutzt werden können, die dem Patienten nicht persönlich zugewiesen sind und die daher auch überhaupt keine Kenntnis davon haben, wer mit ihnen umgeht.

Das gleiche gilt auch für ein Apparatesystem, wie es in der DE 100 54 960 A1 zur Untersuchung des Zustandes und der Bereitschaft für Zusammenarbeit und/oder Therapievorbereitung eines Patienten eingesetzt werden soll. Dort geht es nur um die Zugangsberechtigung zum Zugriff auf die Patientenakte, wofür eine Benutzeridentifikation erforderlich ist. Die Geräte, die benutzt werden, kennen bei dieser Anordnung den jeweils Nutzenden, was bei echten Compliance-Untersuchungen gerade nicht der Fall ist.

Der Erfindung liegt daher die Aufgabe zugrunde, ein Verfahren zur automatisierten Erfassung von Patienten-Aktionen zu schaffen, das eine erheblich höheren Grad an Standardisierung aufweist und das verbreitete Mogeleien bei der Datenerhebung sowohl beim Patienten, als auch beim klinischen Personal im Zuge von klinischen Studien weitestgehend ausschließt.

Zur Lösung dieser Aufgabe ist erfindungsgemäß vorgesehen, dass die hierbei verwendeten medizinischen Geräte oder Einrichtungen mit Erfassungseinrichtungen zur Identifikation des Patienten versehen sind, um die bei der Benutzung der Geräte oder Hilfsmittel anfallenden Daten automatisch dem betreffenden Patienten zuzuordnen. Derartige Identifizierungsgeräte können beispielsweise Chipkarten, Transponder od. dgl. sein, wobei dann die Erfassungseinrichtungen passende Gegenstellen, z. B. Chipkartenleser, Transponder-Gegenstelle od. dgl., sind.

Als Identifizierungsvorrichtung kann auch eine Vorrichtung zur Erkennung biometrischer Merkmale, wie z. B. dem Fingerabdruck, dienen.

Durch die erfindungsgemäße Ausgestaltung ist eine automatische Erfassung dahingehend möglich, welcher Patient z. B. welchen Messwert erzeugt hat, welches Hilfsmittel er benutzt hat oder gegebenenfalls welchen Behandlungsraum er aufgesucht hat, und gleichzeitig der Erfassung des Zeitpunktes, zu dem diese Aktion durchgeführt wurde.

In dieser Art der automatischen Erfassung welcher Patient welchen Messwert erzeugt hat, wobei das Gerät den Patienten vorher überhaupt nicht gekannt hat, liegt auch der entscheidende Unterschied zu den Anordnungen nach den eingangs genannten Druckschriften zum Stand der Technik. Erfindungsgemäß kennt das Gerät oder die jeweilige Einrichtung den Patienten nicht, es geht also nicht um einen Vergleich der Identifikation des Partners mit abgespeicherten Daten, sonder die Erfassungseinrichtungen zur Identifikation dienen dazu, die erzeugten Gerätewerte mit einer Nutzeradresse zu versehen, aufgrund deren dann die Gerätemesswerte in einer Zentrale ausgewertet werden können. Unter dem Begriff "anfallende Daten" ist dabei im Sinne der Erfindung nicht nur ein von einem Gerät erzeugter Messwert sondern beispielsweise auch die Tatsache zu verstehen, dass ein Patient einen bestimmten Raum betreten oder ein bestimmtes Gerät - egal wie - benutzt hat. Auch dies sind relevante Daten im Zusammenhang mit klinischen Studien oder Compliance-Untersuchungen.

Die so erfassten Daten werden außer mit der Identität des Patienten bevorzugt mit einem Zeitstempel versehen und können wahlweise beim Patienten, z. B. auf dessen Chipkarte, an den einzelnen Netzstellen, z. B. in einem an den Chipkartenleser oder die Transponderstelle angeschlossenen Computer, oder schließlich auch zentral, z. B. auf einem mit den einzelnen Erhebungsstellen vernetzten Rechner, gespeichert werden. Dadurch werden einerseits Abschreibfehler (z. B. vom Display eines Messgerätes auf ein Messprotokoll oder vom Messprotokoll in den Erhebungsbogen) als auch (bewusst oder unbewusst) unrichtige Angabe der beteiligten Personen bezüglich Zeitpunkt, Dauer und Art der durchgeführten Aktionen ausgeschlossen.

Die so erhobenen Daten ermöglichen z. B. eine automatische Überprüfung ob ein Patient die Einschlusskriterien für die Auswertung der Studie erfüllt oder ob seine Daten, z. B. wegen zu unregelmäßiger Einnahme der Medikamente, unberücksichtigt bleiben sollten. Dadurch wird die Variabilität der Ergebnisse reduziert und die Aussagekraft der Analyse verbessert. Zudem ist es denkbar, die Teilnehmerzahl zu verringern, weil aufgrund der reduzierten Variabilität signifikante Aussagen leichter, d. h. mit weniger Stichproben, zu erreichen sind. Das beschriebene System eignet sich insbesondere auch zur Überwachung der Compliance von Patienten, die mit neuartigen telemedizinischen Versorgungsformen betreut werden.

In weiterer Ausgestaltung der Erfindung kann dabei vorgesehen sein, dass die abgespeicherten Daten gegen in der Erfassungsvorrichtung, dem angeschlossenen Computer oder dem vernetzten Zentralrechner gespeicherte Soll-Vorgaben verglichen werden und die Patientenaktion als den Soll-Vorgaben entsprechend oder nicht entsprechend gekennzeichnet wird, wobei bevorzugt dann nur als den Soll-Vorgaben entsprechend gekennzeichnete Aktionen in die Datenbank zur Auswertung einer klinischen Studie übernommen werden. Alle den Soll-Vorgaben nicht entsprechende Aktionen im Rahmen einer telemedizinischen Betreuung können mit einem Warnhinweis an den betreuenden medizinischen Leistungserbringer über das angeschlossene Datennetzwerk versandt werden.

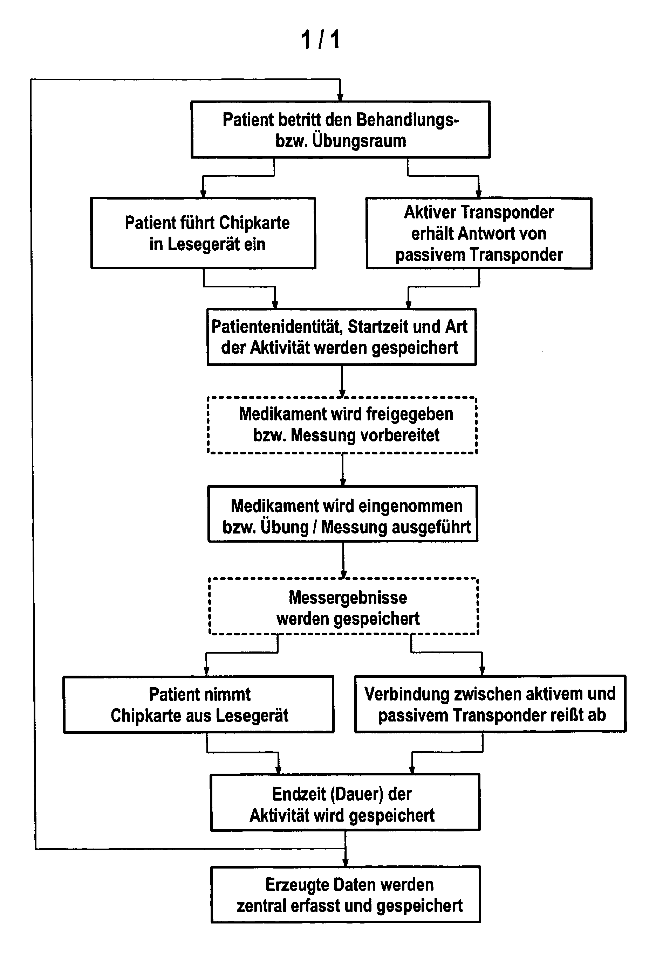

Weitere Vorteile, Merkmale und Einzelheiten der Erfindung ergeben sich aus der nachfolgenden Beschreibung eines Ausführungsbeispiels sowie anhand der Zeichnung, die ein Ablaufdiagramm eines erfindungsgemäßen automatisierten Erfassungssystems zeigt.

Sobald der Patient den Behandlungs- bzw. Übungsraum betritt, führt er entweder sein Identifizierungsgerät, beispielsweise eine Chipkarte, in ein entsprechendes Lesegerät ein oder aber ein aktiver Transponder im Raum oder am Untersuchungsgerät oder Übungsgerät erhält Antwort von passiven Transponder des Identifizierungsgeräts des Patienten. Die Patientenidentität, die Startzeit und die Art der Aktivität werden gespeichert. Gegebenenfalls wird das einzunehmende Medikament freigegeben bzw. eine Messung vorbereitet. Nach der Einnahme des Medikaments und der Durchführung der Übung oder Messung werden die Messergebnisse gespeichert. Beim Verlassen des Behandlungs- bzw. Übungsraums entnimmt der Patient entweder seine Chipkarte aus dem Lesegerät oder aber die Verbindung zwischen aktivem und passivem Transponder reißt ab, was in beiden Fällen als Endzeit (Dauer) der Aktivität abgespeichert wird. Nach lokaler Erfassung und Speicherung der erzeugten Daten werden diese gegebenenfalls über ein Datennetzwerk an eine zentrale Datenbank übermittelt und dort zentral gespeichert.

## Patentansprüche

1. Verfahren zur automatisierten Erfassung von Patienten-Aktionen, beispielsweise im Zuge einer telemedizinischen Versorgung oder im Zuge klinischer Studien zur Güte einer Therapieform oder zur Erprobung der Wirksamkeit von Arzneimitteln, **dadurch gekennzeichnet, dass** die hierbei verwendeten medizinischen Geräte oder Einrichtungen mit Erfassungseinrichtungen zur Identifikation des Patienten versehen sind, um die bei der Benutzung der Geräte oder Hilfsmittel anfallenden Daten automatisch dem betreffenden Patienten zuzuordnen.

2. Verfahren nach Anspruch 1, **dadurch ge-kennzeichnet,dass** die Erfassungseinrichtung zur Identifikation des Patienten Chipkartenleser, Transponder od. dgl. sind, zu welchen der Patient ein individuell ihm zugeordnetes Gegenstück mit sich führt bzw. benutzt, wie z. B. eine Chipkarte oder ein Gegenstück des Transponders.

3. Verfahren nach Anspruch 1 oder 2, **dadurch gekennzeichnet, dass** die Erfassungseinrichtungen zur Identifikation des Patienten biometrische Merkmale, wie z. B. den Fingerabdruck des Patienten, erfassen.

4. Verfahren nach einem der Ansprüche 1 bis 3, **da-durch gekennzeichnet, dass** die anfallenden und dem Patienten zugeordneten Daten in der Erfassungseinrichtung zur Patientenidentifikation, dem Therapiegerät, dem Gerät zur Datenerhebung, dem technischen Hilfsmittel oder der individuellen Chipkarte etc. abgespeichert werden.

5. Verfahren nach einem der Ansprüche 1 bis 4, **dadurch gekennzeichnet, dass** die erhobenen und dem Patienten zugeordneten Daten an der Datenerhebungsstelle, vorzugsweise in einem an die Erfassungseinrichtung angeschlossenen, Computer gespeichert werden.

6. Verfahren nach einem der Ansprüche 1 bis 5, **dadurch gekennzeichnet, dass** die erhobenen und dem Patienten zugeordneten Daten auf einem mit verschiedenen Erhebungsstellen vernetzten Zentralrechner gespeichert werden.

7. Verfahren nach einem der Ansprüche 1 bis 6, **dadurch gekennzeichnet, dass** mit den erhobenen Daten auch der Erhebungsort und/oder das Erhebungsgerät und/oder der Beginn des Erhebungszeitpunktes und/oder das Ende des Erhebungszeitpunktes mit erfasst und abgespeichert werden.

8. Verfahren nach einem der Ansprüche 1 bis 7, **dadurch gekennzeichnet, dass** die Erfassungseinrichtungen geräteseitig oder patientenseitig eine Vorrichtung zur Bestimmung des Ortes, wie z. B. ein GPS-System, enthält.

9. Verfahren nach einem der Ansprüche 6 bis 8, **dadurch gekennzeichnet, dass** die abgespeicherten Daten gegen in der Erfassungsvorrichtung, dem angeschlossenen Computer oder dem vernetzten Zentralrechner gespeicherte Sollvorgaben verglichen werden, und die Patientenaktion als den Sollvorgaben entsprechend oder nicht entsprechend gekennzeichnet wird.

10. Verfahren nach Anspruch 9, **dadurch gekennzeichnet,dass** nur als den Sollvorgaben entsprechend gekennzeichnete Aktionen in die Datenbank zur Auswertung einer klinischen Studie übernommen werden.

11. Verfahren nach Anspruch 9 oder 10, **dadurch gekennzeichnet, dass** bei den Sollvorgaben nicht entsprechenden Aktionen im Rahmen einer telemedizinischen Betreuung ein Warnhinweis an den betreuenden medizinischen Leistungserbringer über das angeschlossene Datennetzwerk versandt wird.
